# FASCICULE DE BREVET EUROPEEN

(11) **EP 2 503 964 B1**
(45) Date de publication et mention de la délivrance du brevet: **21.08.2013**
(21) Numéro de dépôt: 10734281.8
(22) Date de dépôt: 21.06.2010
(51) Int. Cl.: A61F 5/02

(54) **CEINTURE DE SOUTIEN LOMBAIRE**
LUMBALER STÜTZGURT
LUMBAR SUPPORT BELT

(30) Priorité: 25.11.2009 FR 0958380
(43) Date de publication de la demande: 03.10.2012
(73) Titulaire: Gibaud, 42000 Saint Etienne (FR)
(72) Inventeur: ANGLADA, Gérard, F-42000 Saint Etienne (FR); BECKERS, Thomas, F-69630 Chaponost (FR)
(74) Mandataire: Delorme, Nicolas
(86) Numéro de dépôt international: PCT/FR2010/051243
(87) Numéro de publication internationale: WO 2011/064476

(56) Documents cités:
- WO-A1-03/017893
- WO-A1-2009/113053
- US-A- 2 543 095
- US-A- 4 622 957
- US-A- 4 836 194

## Description

La présente invention concerne une ceinture de soutien lombaire.

Une telle ceinture est destinée aux personnes souffrant de lombalgie, notamment, et comprend classiquement une bande de textile élastique destinée à être positionnée autour de la partie inférieure du tronc d'une personne. Cette bande présente une partie centrale ainsi qu'une première et une deuxième parties extrêmes qui sont pourvues de moyens d'accrochage complémentaires et qui sont destinées à être placées l'une sur l'autre en regard de la zone abdominale de la personne et à être assemblées par lesdits moyens d'accrochage.

Ainsi positionnée, la ceinture permet d'une part de diminuer la douleur ressentie par le porteur et d'autre part de maintenir le porteur dans une bonne position, en particulier grâce à la contention de la région lombaire et de la région abdominale du porteur.

En pratique, on constate que la ceinture occasionne une charge importante sur les vertèbres basses, c'est-à-dire en particulier les quatrième et cinquième vertèbres lombaires (L4 - L5). Ceci, outre la douleur ressentie par le porteur, n'est pas pleinement satisfaisant d'un point de vue orthopédique.

En outre, lorsque la ceinture est serrée autour de la partie inférieure du tronc d'une personne, la bande, étant tendue, ne peut pas épouser la courbure lombaire et, en conséquence, ne peut pas exercer un support efficace de cette zone. Or c'est principalement cette zone de la colonne qui nécessite un soutien important.

Les dispositifs orthopédiques connus permettant de résoudre les problèmes précités se présentent sous la forme de corsets relativement rigides, réalisés sur mesure, et pourvus de moyens de rigidification tels que des baleines adaptés à la morphologie du patient. Ces dispositifs sont donc coûteux et généralement lourds et peu confortables.

Les documents US 4 622 957, WO 03/017893, US 2 543 095, US 4 836 194 et WO 2009/113053 décrivent des ceintures pouvant être équipées de coussins. Une ceinture de soutien lombaire selon le préambule de la revendication 1 est notamment décrite dans le document US 4 622 957.

Il existe donc un besoin d'une ceinture de soutien lombaire dont l'efficacité et le confort de port soient améliorés, et qui puisse remédier aux inconvénients mentionnés ci-dessus. Dans ce contexte, l'invention concerne une ceinture de soutien lombaire comprenant :
- une bande de textile élastique destinée à être positionnée autour de la partie inférieure du tronc d'une personne, ladite bande présentant un axe médian transversal et possédant une partie centrale ainsi qu'une première et une deuxième parties extrêmes qui, pourvues de moyens d'accrochage complémentaires, sont destinées à être placées l'une sur l'autre en regard de la zone abdominale de la personne et à être assemblées par lesdits moyens d'accrochage ;
- et un coussin fixé sur la face intérieure de la partie centrale de la bande, de façon sensiblement centrée par rapport à l'axe médian transversal de la bande.

Selon une définition générale de l'invention, le coussin présente sensiblement la forme d'un V dont la pointe est dirigée vers le bas lorsque la ceinture est portée et possédant deux ailes reliées par une zone de jonction, le coussin étant conçu et disposé pour que, lorsque la ceinture est portée, la partie extrême libre de chaque aile prenne appui sur la crête iliaque correspondante de la personne et la zone de jonction soit en contact avec la zone comprenant la partie haute du sacrum et la partie basse des vertèbres lombaires de la personne.

En créant un appui sur les crêtes iliaques, c'est-à-dire en déportant l'appui de la zone lombaire vers les crêtes iliaques, la ceinture permet de diminuer la pression mécanique exercée par le haut du corps sur les vertèbres basses. En déchargeant ainsi les vertèbres basses, par appui sur des zones dites molles, on crée une sorte d'élongation de la colonne vertébrale qui est très bénéfique en termes de contention. Les ailes du coussin constituent de plus un repère pour la personne mettant la ceinture car en plaçant ces ailes sur les hanches, la personne s'assure du bon positionnement de la ceinture.

De plus, en prévoyant, grâce à la zone de jonction du coussin, un contact sensiblement au niveau du haut du pli fessier, dans la zone de la cinquième vertèbre lombaire et du haut du sacrum (L5 - S1), la ceinture assure un appui dans l'une des zones les plus fragiles de la colonne.

La ceinture selon l'invention permet de combiner l'effet de deux appuis et de créer sensiblement une continuité d'appui, surtout pour une personne ayant une lordose prononcée. De ce fait, l'efficacité mécanique de la ceinture est fortement augmenté, sans qu'il soit nécessaire pour cela de serrer la bande de façon importante. Le confort de port s'en trouve donc amélioré.

En outre, le coussin, et plus particulièrement la zone de jonction du coussin, contribue à combler la zone de vacuité située dans la courbure lombaire. Ceci permet d'améliorer le contact entre la bande et le porteur dans cette zone et donc le soutien conféré par la ceinture. Ce comblement étant obtenu par le coussin, qui est un élément souple, et non par des éléments rigides mis en forme, comme des baleines, la ceinture peut conserver ses avantages en termes de contact et de support quelle que soit la position du porteur. Le coussin permet donc de bien adapter la ceinture à l'anatomie de la personne tout en garantissant la respirabilité de la ceinture par un choix approprié du matériau employé.

Afin d'améliorer le comblement de la lordose du porteur, on peut prévoir en outre que le coussin comporte un appendice s'étendant depuis la zone de jonction vers le haut, lorsque la ceinture est portée, sensiblement le long de l'axe médian transversal de la bande.

De préférence, la zone située entre les ailes du coussin - que l'appendice soit présent ou non - n'est pas entièrement comblée.

Selon une réalisation possible, vu en coupe dans un plan orthogonal à la bande et passant par l'axe médian transversal, l'appendice présente une forme bombée agencée pour combler au moins partiellement la lordose de la personne portant la ceinture.

Les ailes du coussin peuvent être incurvées vers l'intérieur du V, pour mieux s'adapter à la morphologie du porteur, notamment aux hanches.

Lorsque la ceinture est portée par une personne, l'extrémité libre des ailes du coussin est par exemple située au voisinage de chacun des flancs de la personne. En variante, l'extrémité libre des ailes du coussin est située dans la région latérale de l'abdomen de la personne.

Avantageusement, le coussin peut être fixé à la bande de façon amovible. De la sorte, il est possible de détacher le coussin pour le laver, ou si la personne souhaite porter la bande seule, sans coussin. A cet effet, le coussin peut comprendre trois zones d'accrochage situées à l'extrémité libre des ailes et dans la zone de jonction, la face intérieure de la bande comportant trois zones d'accrochage localisées complémentaires. Il peut s'agir de moyens agrippants tels que des boucles et des crochets d'un système Velcro ®. En prévoyant un coussin d'une seule pièce muni de zones d'accrochage localisées, on facilite le placement correct du coussin sur la bande, sans interprétation possible du porteur.

En variante, on peut prévoir une fixation inamovible du coussin sur la bande, typiquement par couture.

Par ailleurs, la ceinture peut comprendre au moins une sangle de serrage additionnel. Cette caractéristique est particulièrement avantageuse en combinaison avec le coussin selon l'invention. En effet, les ceintures existent en un nombre de tailles restreint. Suivant que le porteur de la ceinture se situe dans le haut ou dans le bas de la plage anatomique couverte par la taille de sa ceinture, il ne pourra obtenir exactement la contention souhaitée avec la bande seule. La ou les sangles de serrage additionnel permettent de mieux régler le serrage et donc d'améliorer l'appui obtenu avec le coussin, ce qui se traduit favorablement sur l'efficacité de la ceinture.

On décrit à présent, à titre d'exemples non limitatifs, plusieurs modes de réalisation possibles de l'invention, en référence aux figures annexées :
La figure 1 est une vue en plan de la face intérieure d'une ceinture selon un premier mode de réalisation l'invention ;
Les figures 2 à 4 représentent de façon schématique la ceinture de la figure 1 mise en place sur une personne vue respectivement de dos, de côté et de face ;
La figure 5 est une vue en plan de la face intérieure d'une ceinture selon un deuxième mode de réalisation l'invention ;
La figure 6 montre une personne vue de dos, sans la ceinture ;
Les figures 7 et 8 représentent de façon schématique la ceinture de la figure 5 mise en place sur une personne vue respectivement de dos et de côté ;
Les figures 9 et 10 sont des vues schématiques d'une personne portant la ceinture de la figure 5, en coupe horizontale, respectivement dans un plan passant par les vertèbres L4/L5 (ligne I des figures 6 à 8) et par la vertèbre L3 (ligne II des figures 6 à 8).

La ceinture 1 selon l'invention comprend une bande 2 qui est réalisée en un textile élastique et possède une face intérieure 3 - tournée vers le corps du porteur lorsque la bande est en position d'utilisation - et une face extérieure 4.

La bande 2 comprend une partie centrale 5 prolongée par une première et une deuxième parties extrêmes 6, 7 symétriques. La partie centrale 5, qui est élargie par rapport aux parties extrêmes 6, 7, est destinée à venir couvrir la région lombaire du porteur. Elle peut présenter des baleines (non représentées) sur sa face extérieure 4. Par exemple, ces baleines sont réparties en deux groupes de deux baleines parallèles, et forment un V convergeant vers le bas et vers l'axe médian transversal de la bande 2. Les baleines peuvent être logées dans des goussets et présenter une courbure concave venant se loger dans la concavité de la région lombaire du porteur.

La bande 2 est destinée à être enroulée autour de la partie inférieure du tronc d'une personne, les parties extrêmes 6, 7 étant placées l'une sur l'autre en regard de la zone abdominale de la personne. Afin de maintenir la bande dans cette position, la première partie extrême 6 comporte, sur sa face intérieure 3, une zone 9 de moyens d'accrochage agrippants du type crochets d'un système Velcro ® et la deuxième partie extrême 7 comporte, sur sa face extérieure 3, une zone 10 complémentaire, par exemple une zone de type boucles d'un système Velcro ®. L'accrochage de la bande 2 s'effectue alors en plaçant la première partie extrême 6 sur la deuxième partie extrême 7. Toutefois, la structure inverse est également possible.

La bande 2 présente un axe médian transversal 12 - sensiblement vertical lorsque la ceinture 1 est portée - et un axe médian longitudinal 13, sensiblement orthogonal à l'axe médian transversal et situé sensiblement à mi hauteur de la partie centrale 5.

La ceinture 1 comprend de plus un coussin 14 fixé sur la face intérieure 4 de la partie centrale 5 de la bande 2, de façon sensiblement centrée par rapport à l'axe médian transversal 12 de la bande 2. Le coussin 14 est réalisé en un matériau souple lui permettant d'épouser la forme du corps de la personne, et de préférence en un matériau respirant.

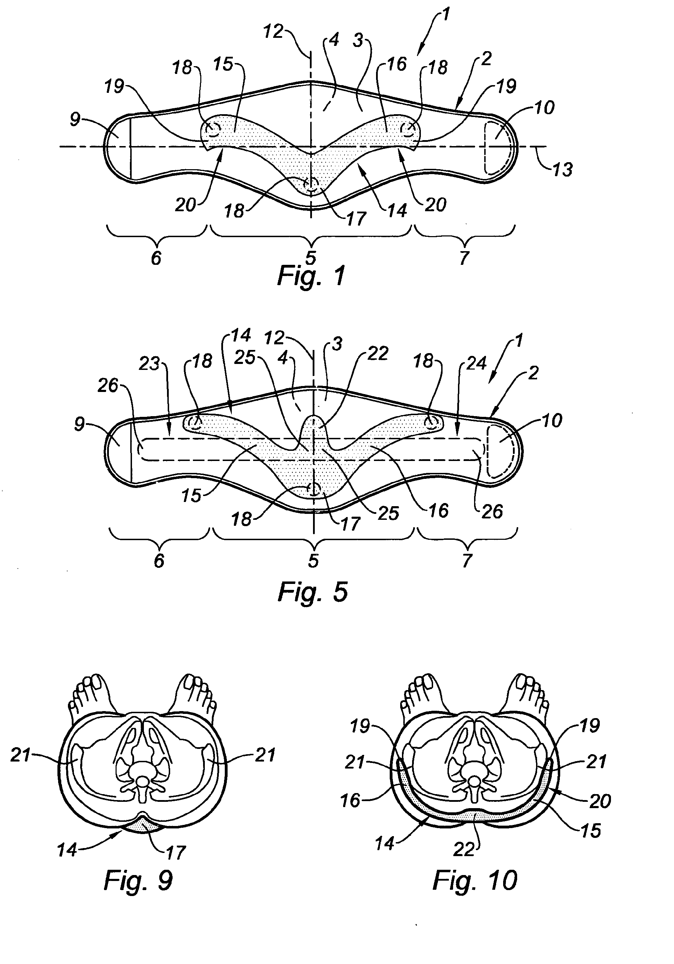

Selon un premier mode de réalisation, illustré sur les figures 1 à 4, le coussin 14 présente sensiblement la forme d'un V évasé dont la pointe est dirigée vers le bas - lorsque la ceinture 1 est portée. Le coussin 14 possède deux ailes 15, 16 de forme incurvée reliées par une zone de jonction 17, au niveau de la pointe du V. L'angle formé entre les ailes 15, 16 est par exemple compris entre 100° et 150°, notamment de l'ordre de 120°.

Dans la réalisation représentée, le coussin 14 est fixé à la bande 2 de façon amovible. A cet effet, le coussin 14 possède sur sa face tournée vers la bande 2 trois zones d'accrochage 18 situées à l'extrémité libre 19 des ailes et dans la zone de jonction 17, tandis que la bande 2 possède, sur sa face intérieure 4, trois zones d'accrochage localisées complémentaires.

Les zones d'accrochage de la bande 2 sont ménagées de sorte que, lorsque le coussin 14 est fixé sur la bande 2 et que la ceinture 1 est portée par une personne dans la position appropriée :
- d'une part la partie extrême libre 20 de chaque aile 15, 16 prend appui sur la crête iliaque 21 correspondante de la personne ;
- et d'autre part la zone de jonction 17 est en contact avec la zone de la cinquième vertèbre lombaire (L5) et du haut du sacrum S (S1) de la personne.

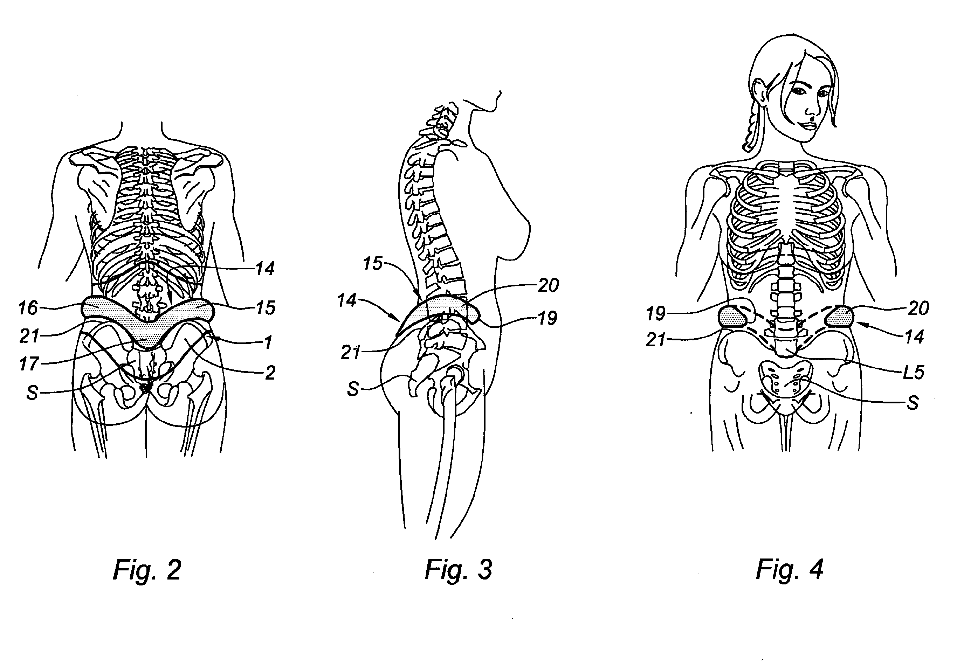

De plus, comme on le voit sur la figure 4, l'extrémité libre 19 des ailes 15, 16 du coussin 14 est située dans la région latérale de l'abdomen de la personne.

La ceinture 1 peut en outre comporter un dispositif de serrage additionnel (non représenté).

Selon un deuxième mode de réalisation, illustré sur les figures 5 à 10, le coussin 14 présente sensiblement la même forme que précédemment décrite et comporte en outre un appendice 22. L'appendice 22 s'étend depuis la zone de jonction 17 vers le haut, lorsque la ceinture 1 est portée, sensiblement le long de l'axe médian transversal 12 de la bande 2. De préférence, l'appendice 22 présente une forme bombée vu en coupe dans un plan orthogonal à la bande 2 et passant par l'axe médian transversal 12. De la sorte, on obtient un meilleur comblement de la courbure lombaire de la personne portant la ceinture 1.

Comme illustré schématiquement sur la figure 5, la ceinture 1 peut en outre comporter un dispositif de serrage additionnel pouvant notamment être utilisé quand le porteur doit faire un effort ponctuel. Ce dispositif comprend ici deux sangles latérales 23, 24 de serrage additionnel. Chaque sangle 23, 24 comprend une première extrémité 25 fixée sur la face extérieure 4 de la partie centrale 5 de la bande 2. La deuxième extrémité 26 - libre - de chaque sangle 23, 24 comprend, sur sa face intérieure, des moyens d'accrochage sur l'autre sangle et/ou sur la bande 2.

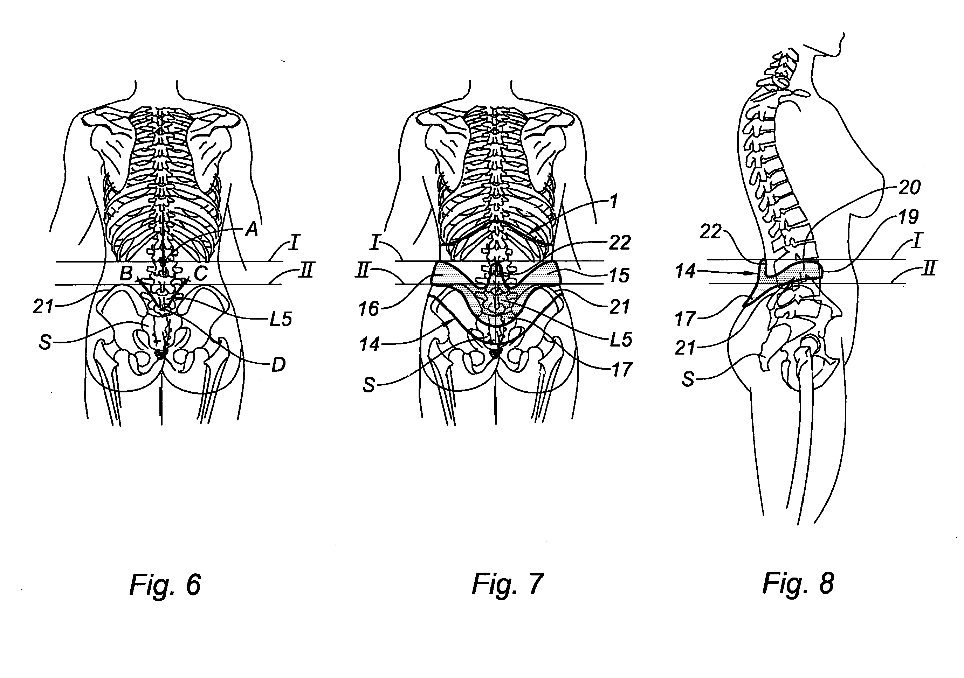

Comme pour le premier mode de réalisation, le coussin 14 est fixé à la bande 2 de façon amovible et comporte à cet effet les trois zones d'accrochage 18 décrites précédemment. Ainsi, comme illustré sur les figures 6 à 10, lorsque le coussin 14 est fixé sur la bande 2 et que la ceinture 1 est portée par une personne dans la position appropriée :
- la partie extrême libre 20 de chaque aile 15, 16 prend appui sur la crête iliaque 21 correspondante de la personne ;
- la zone de jonction 17 est en contact avec la zone de la cinquième vertèbre lombaire (L5) et du haut du sacrum S (S1) de la personne ;
- l'appendice 22 est en contact avec la région lombaire de la personne, en épousant et en comblant au moins partiellement la lordose ;
- l'extrémité libre 19 des ailes 15, 16 du coussin 14 est située au voisinage de chacun des flancs de la personne (voir figure 8), c'est-à-dire que, dans la réalisation représentée qui n'et pas limitative, les ailes 15, 16 ne s'étendent pas jusqu'à la région latérale de l'abdomen de la personne.

Ainsi, l'invention apporte une amélioration déterminante à la technique antérieure, en fournissant une ceinture dont l'efficacité et le confort de port sont nettement améliorés.

Le coussin 14, associé à la bande 2 permet de réaliser un appui et un comblement dans tout ou partie de la zone du losange de Michaelis, qui est le siège des douleurs lombaires les plus fréquentes. Comme représenté sur la figure 6, ce losange est délimité par quatre sommets, à savoir le bas du sillon lombaire A, les deux fossettes sacro iliaques B, C et le sommet D du sillon inter fessier.

Le coussin 14 permet de maintenir en permanence la pression antalgique de la ceinture dans les zones naturelles de vacuité : lordose lombaire, bas du sillon lombaire et haut du sillon inter fessier ; Les ailes du coussin qui prennent appui sur les crêtes iliaques permettent d'augmenter la pression dans la zone molle pour améliorer la décharge des vertèbres basses.

Il va de soi que l'invention n'est pas limitée aux modes de réalisation décrits ci-dessus à titre d'exemples mais qu'elle en embrasse au contraire toutes les variantes de réalisation.

## Revendications

1. Ceinture de soutien lombaire comprenant :
- une bande (2) de textile élastique destinée à être positionnée autour de la partie inférieure du tronc d'une personne, ladite bande (2) présentant un axe médian transversal (12) et possédant une partie centrale (5) ainsi qu'une première et une deuxième parties extrêmes (6, 7) qui, pourvues de moyens d'accrochage complémentaires (9, 10), sont destinées à être placées l'une sur l'autre en regard de la zone abdominale de la personne et à être assemblées par lesdits moyens d'accrochage ;
- un coussin (14) fixé sur la face intérieure (3) de la partie centrale (5) de la bande (2), de façon sensiblement centrée par rapport à l'axe médian transversal (12) de la bande (2) ;
**caractérisée en ce que** le coussin (14) présente sensiblement la forme d'un V dont la pointe est dirigée vers le bas lorsque la ceinture (1) est portée et possédant deux ailes (15, 16) reliées par une zone de jonction (17), le coussin (14) étant conçu et disposé pour que, lorsque la ceinture (1) est portée, la partie extrême libre (20) de chaque aile (15, 16) prenne appui sur la crête iliaque (21) correspondante de la personne et la zone de jonction (17) soit en contact avec la zone comprenant la partie haute du sacrum et la partie basse des vertèbres lombaires de la personne.

2. Ceinture selon la revendication 1, **caractérisée en ce que** le coussin (14) comporte en outre un appendice (22) s'étendant depuis la zone de jonction (17) vers le haut, lorsque la ceinture (1) est portée, sensiblement le long de l'axe médian transversal (12) de la bande (2).

3. Ceinture selon la revendication 2, **caractérisée en ce que**, vu en coupe dans un plan orthogonal à la bande (2) et passant par l'axe médian transversal (12), l'appendice (22) présente une forme bombée agencée pour combler au moins partiellement la lordose de la personne portant la ceinture (1).

4. Ceinture selon l'une des revendications 1 à 3, **caractérisée en ce que** les ailes (15, 16) du coussin (14) sont incurvées vers l'intérieur du V.

5. Ceinture selon l'une des revendications 1 à 4, **caractérisée en ce que**, lorsque la ceinture (1) est portée par une personne, l'extrémité libre (19) des ailes (15, 16) du coussin (14) est située au voisinage de chacun des flancs de la personne.

6. Ceinture selon l'une des revendications 1 à 4, **caractérisée en ce que**, lorsque la ceinture (1) est portée par une personne, l'extrémité libre (19) des ailes (15, 16) du coussin (14) est située dans la région latérale de l'abdomen de la personne.

7. Ceinture selon l'une des revendications 1 à 6, **caractérisée en ce que** le coussin (14) est fixé à la bande (2) de façon amovible.

8. Ceinture selon la revendication 7, **caractérisée en ce que** le coussin (14) comprend trois zones d'accrochage (18) situées à l'extrémité libre (19) des ailes (15, 16) et dans la zone de jonction (17) et **en ce que** la face intérieure (3) de la bande (2) comporte trois zones d'accrochage localisées complémentaires.

9. Ceinture selon l'une des revendications 1 à 8, **caractérisée en ce qu'**elle comprend au moins une sangle de serrage additionnel (23, 24).

## Patentansprüche

1. Lumbaler Stützgurt, umfassend:
- ein Band (2) aus elastischem Stoff, dazu ausgelegt, um um den unteren Teil des Rumpfes einer Person angebracht zu werden, wobei das Band (2) eine quer liegende Mittelachse (12) aufweist und einen zentralen Teil (5) umfasst, ebenso wie einen ersten und zweiten Endteil (6, 7), die, versehen mit zusätzlichen Befestigungsmitteln (9, 10), ausgelegt sind, um mit Bezug auf den Unterleibsbereich der Person aufeinander angebracht und durch die Befestigungsmittel montiert zu werden.
- ein Kissen (14), das auf der inneren Seite (3) des zentralen Teils (5) des Bandes (2) auf eine im Wesentlichen zentrierte Weise mit Bezug auf die quer liegende Mittelachse (12) des Bandes (2) befestigt ist;
**dadurch gekennzeichnet, dass** das Kissen (14) im Wesentlichen die Form eines V aufweist, dessen Spitze nach unten gerichtet ist, wenn der Gurt (1) getragen wird, und zwei Flügel (15, 16) umfasst, die durch einen Verbindungsbereich (17) miteinander verbunden sind, wobei das Kissen (14) entworfen und angebracht ist, damit, wenn der Gurt (1) getragen wird, der freie Endteil (20) jedes Flügels (15, 16) auf dem entsprechenden Beckenkamm (21) der Person aufliegt und der Verbindungsbereich (17) mit dem Bereich in Kontakt steht, der den oberen Teil des Kreuzbeins und den unteren Teil der Lendenwirbel der Person umfasst.

2. Gurt nach Anspruch 1, **dadurch gekennzeichnet, dass** das Kissen (14) außerdem ein Ansatzstück (22) umfasst, das sich vom Verbindungsbereich (17) nach oben erstreckt, wenn der Gurt (1) getragen wird, im Wesentlichen entlang der quer liegenden Mittelachse (12) des Bandes (2).

3. Gurt nach Anspruch 2, **dadurch gekennzeichnet, dass** gesehen im Schnitt in einer orthogonalen Ebene des Bandes (2) und passierend durch die quer liegende Mittelachse (12) das Ansatzstück (22) eine gewölbte Form aufweist, die angeordnet ist, um mindestens teilweise die Rückgratverkrümmung der Person, die den Gurt trägt, auszugleichen.

4. Gurt nach einem der Ansprüche 1 bis 3, **dadurch gekennzeichnet, dass** die Flügel (15, 16) des Kissens (14) auf die Innenseite des V hin gebogen sind.

5. Gurt nach einem der Ansprüche 1 bis 4, **dadurch gekennzeichnet, dass** wenn der Gurt (1) von einer Person getragen wird, sich das freie Ende (19) der Flügel (15, 16) des Kissens (15) neben jeder der Seiten der Person befindet.

6. Gurt nach einem der Ansprüche 1 bis 4, **dadurch gekennzeichnet, dass** wenn der Gurt (1) von einer Person getragen wird, sich das freie Ende (19) der Flügel (15, 16) des Kissens (14) im seitlichen Bereich des Unterleibs der Person befindet.

7. Gurt nach einem der Ansprüche 1 bis 6, **dadurch gekennzeichnet, dass** das Kissen (14) auf abnehmbare Weise mit dem Band (2) verbunden ist.

8. Gurt nach Anspruch 7, **dadurch gekennzeichnet, dass** das Kissen (14) drei Befestigungsbereiche (18) aufweist, die sich am freien Ende (19) der Flügel (15, 16) und im Verbindungsbereich (19) befinden, und dadurch, dass die innere Seite (3) des Bands (2) drei Befestigungsbereiche umfasst, die zusätzlich angebracht sind.

9. Gurt nach einem der Ansprüche 1 bis 8, **dadurch gekennzeichnet, dass** er mindestens einen zusätzlichen Spanngurt (23, 24) umfasst.

## Claims

1. A lumbar support belt, comprising:
- an elastic textile strip (2) designed to be positioned around the lower portion of the torso of a person, said strip (2) having a transverse median axis (12) and having a central portion (5) as well as first and second end portions (6, 7) which, provided with complimentary fastening means (9, 10), are designed to be placed on one another facing the abdominal area of the person and to be assembled using said fastening means;
- a cushion (14) fastened on the inner surface (3) of the central portion (5) of the strip (2), substantially centered with respect to the transverse median axis (12) of the strip (2);
**characterized in that** the cushion (14) is substantially in the shape of a V whereof the tip is oriented downward when the belt (1) is worn and having two wings (15, 16) connected by a junction area (17), the cushion (14) being designed and positioned so that, when the belt (1) is worn, the free end portion (20) of each wing (15, 16) bears on the corresponding iliac crest (21) of the person and the junction area (17) is in contact with the area comprising the upper portion of the sacrum and the lower portion of the lumbar vertebrae of the person.

2. The belt according to claim 1, **characterized in that** the cushion (14) further includes an appendage (22) extending from the junction area (17) upward, when the belt (1) is worn, substantially along the transverse median axis (12) of the strip (2).

3. The belt according to claim 2, **characterized in that**, seen in cross-section in a plane orthogonal to the strip (2) and passing through the transverse median axis (12), the appendage (22) has a curved shape arranged to at least partially fill in the lordosis of the person wearing the belt (1).

4. The belt according to one of claims 1 to 3, **characterized in that** the wings (15, 16) of the cushion (14) are curved toward the inside of the V.

5. The belt according to one of claims 1 to 4, **characterized in that**, when the belt (1) is worn by a person, the free end (19) of the wings (15, 16) of the cushion (14) is situated in the vicinity of each of the person's sides.

6. The belt according to one of claims 1 to 4, **characterized in that**, when the belt (1) is worn by a person, the free end (19) of the wings (15, 16) of the cushion (14) is situated in the lateral region of the person's abdomen.

7. The belt according to one of claims 1 to 6, **characterized in that** the cushion (14) is fastened to the strip (2) removably.

8. The belt according to claim 7, **characterized in** the cushion (14) comprises three fastening areas (18) situated at the free end (19) of the wings (15, 16) and in the junction area (17) and **in that** the inner face (3) of the strip (2) includes three complementary localized fastening areas.

9. The belt according to one of claims 1 to 8, **characterized in that** it comprises at least one additional tightening strap (23, 24).
